(19) Europäisches Patentamt
European Patent Office
Office européen des brevets

(11) Publication number: **0 502 465 A1**

(12) **EUROPEAN PATENT APPLICATION**

(21) Application number: **92103594.5**

(51) Int. Cl.[5]: **C07D 477/00**, A61K 31/40

(22) Date of filing: **02.03.92**

(30) Priority: **07.03.91 GB 9104769**

(43) Date of publication of application: **09.09.92 Bulletin 92/37**

(84) Designated Contracting States: **AT BE CH DE DK ES FR GB GR IT LI LU MC NL PT SE**

(71) Applicant: **GLAXO S.p.A.**
**Via A. Fleming, 2**
**Verona(IT)**

(72) Inventor: **Gaviraghi, Giovanni, Glaxo Spa**
**Via Alessandro Fleming 2**
**I-37100 Verona(IT)**
Inventor: **Donati, Daniele, Glaxo Spa**
**Via Alessandro Fleming 2**
**I-37100 Verona(IT)**
Inventor: **Tarzia, Giorgio, Glaxo Spa**
**Via Alessandro Fleming 2**
**I-37100 Verona(IT)**
Inventor: **Perboni, Alcide, Glaxo Spa**
**Via Alessandro Fleming 2**
**I-37100 Verona(IT)**
Inventor: **Ursini, Antonella, Glaxo Spa**
**Via Alessandro Fleming 2**
**I-37100 Verona(IT)**

(74) Representative: **Brewer, Christopher Laurence et al**
**Glaxo Holdings p.l.c. Glaxo House Berkeley Avenue**
**Greenford, Middlesex UB6 ONN(GB)**

(54) **Esters of antibacterial condensed carbapenemederivatives.**

(57) Compounds of formula (I)

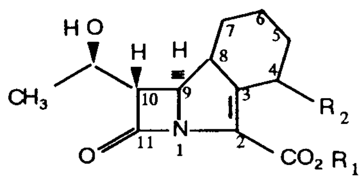

(I)

in which $R_1$ represents the group

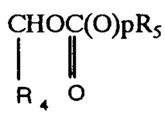

wherein $R_4$ represents a hydrogen atom or a $C_{1-4}$alkyl group; p is zero or one, and $R_5$ represents a group selected from $C_{1-6}$alkyl, $C_{5-8}$cycloalkyl optionally substituted by a $C_{1-3}$alkyl group, phenyl, or $C_{1-4}$alkyl substituted by a $C_{1-3}$alkoxy group.
$R_2$ represents a $C_{1-3}$ alkyl, hydroxy, or hydroxymethyl group or $R_2$ represents the group $XR_3$ wherein X is oxygen and $R_3$ represents a $C_{3-7}$ cycloalkyl or phenyl group or X is the group S(O)n wherein n is zero or the integer 1 or 2 and $R_3$ is $C_{1-5}$alkyl, $C_{3-7}$cycloalkyl or phenyl are orally administrable antibacterial agents.

EP 0 502 465 A1

Rank Xerox (UK) Business Services

This invention relates to heterocyclic derivatives having antibacterial activity, to processes for their preparation to compositions containing them and to their use in medicine.

Thus the present invention provides compounds of the general formula (I)

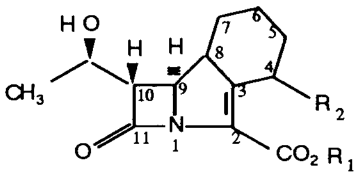

(I)

in which $R_1$ represents the group

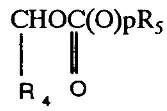

wherein $R_4$ represents a hydrogen atom or a $C_{1-4}$alkyl group; p is zero or one, and $R_5$ represents a group selected from $C_{1-6}$alkyl, $C_{5-8}$cycloalkyl optionally substituted by a $C_{1-3}$alkyl group, phenyl, or $C_{1-4}$alkyl substituted by a $C_{1-3}$alkoxy group.

$R_2$ represents a $C_{1-3}$ alkyl, hydroxy, or hydroxymethyl group or $R_2$ represents the group $XR_3$ wherein X is oxygen and $R_3$ represents a $C_{3-7}$ cycloalkyl or phenyl group or X is the group S(O)n wherein n is zero or the integer 1 or 2 and $R_3$ is $C_{1-5}$alkyl, $C_{3-7}$cycloalkyl or phenyl.

In addition to the fixed stereochemical arrangement as defined in formula (I) the molecule contains a further asymmetric carbon atoms at the 8 and another at the 4 position. Also the group $R_1$ contains at least one asymmetric carbon atom when $R_4$ is other than hydrogen. It will be appreciated that all stereoisomers including mixtures thereof arising from these additional asymmetric centres, are within the scope of the compounds of formula (I).

The general formula (I) as drawn includes at least 4 stereoisomers and mixtures thereof and these may be represented by the formulae (1a, 1b, 1c and 1d).

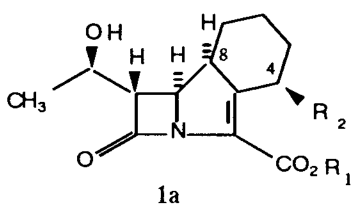

1a

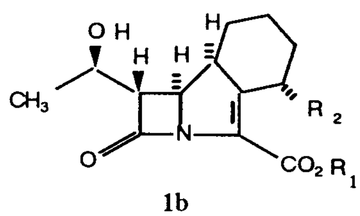

1b

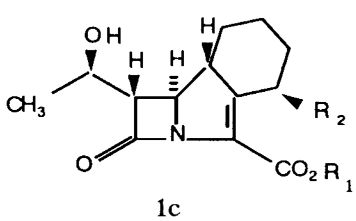

1c

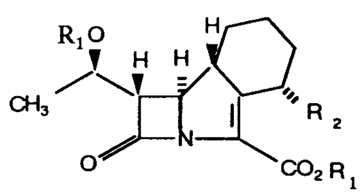

1d

The wedge shaped bond  indicates that the bond is above the plane of the paper. The broken bond

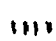

indicates that the bond is below the plane of the paper.

The configuration shown for the carbon atom at the 8-position in formulae 1a and 1b is hereinafter referred to as the $\beta$-configurationand in formulae 1c and 1d as the $\alpha$- configuration.

The configuration shown for the carbon at the 4-position in formulae 1b and 1d is hereinafter referred to as the α-configuration and in formulae 1a and 1c as the β configuration.

In general, in the specific compounds named below, the β-configuration at the 8-position corresponds to the S isomer and the β-configuration at the 4-position to the R isomer. The α-configuration at the 8-position corresponds to the R isomer and the α- configuration at the 4-position corresponds to the S isomer. The assignment of the R or S configuration at the 4- and 8-positions has been made according to the rules of Cahn. Ingold and Prelog, Experientia 1956, 12, 81.

The term alkyl as usual herein refers to a straight or branched chain alkyl group. When $R_4$ represents a $C_{1-4}$ alkyl group this may be for example methyl, ethyl, propyl, isopropyl or butyl.

When $R_5$ represents an alkyl group this may conveniently be a $C_{1-4}$alkyl group such as methyl, ethyl, isopropyl or t-butyl.

When $R_5$ represents a $C_{1-4}$alkyl group substitued by $C_{1-3}$alkoxy, this may be for example a methyl, ethyl, propyl or isopropyl group substituted by methoxy.

When $R_5$ represents $C_{5-8}$cycloalkyl optionally substituted by $C_{1-3}$alkyl this may be for example a cyclopentyl, cyclohexyl, cycloheptyl or cyclooctyl group optionally substitued by a methyl or ethyl group.

When $R_2$ represents the group S(O)n$R_3$, n is preferably zero or one and within this group $R_3$ is preferably $C_{1-5}$alkyl e.g. methyl or phenyl.

A preferred class of compounds of formula (1) are those having the β-configuration at the 8-position. Within this class those having the α-configuration at the 4-position are particularly preferred.

A further preferred class of compounds of formula (I) are those wherein $R_4$ represents, hydrogen, methyl, propyl isopropyl, more particularly hydrogen or methyl.

Yet a further preferred class of compounds of formula (I) are those wherein $R_5$ represents a $C_{1-4}$alkyl group such as methyl, ethyl, isopropyl or t-butyl, or a $C_{1-4}$alkyl group substituted by methoxy such as 1-methoxy-1-methylethyl, or phenyl or a $C_{5-6}$ cycloalkyl group such as cyclopentyl or cyclohexyl optionally substituted by a methyl or ethyl group e.g. ethylcyclohexyl.

Compounds of formula (I) wherein $R_2$ is a hydroxy, hydroxymethyl, phenythio, alkylthio group such as methylthio or methylsulphinyl, represent a further preferred class of compound according to the invention.

A particularly preferred group of esters according to the invention are those wherein $R_4$ represents a hydrogen atom or a methyl group, p is zero or 1 and $R_5$ represents a methyl, ethyl, isopropyl, t-butyl, 1-methoxy-1-methylethyl, phenyl, cyclohexyl, or 4-ethylcyclohexyl group.

A specific preferred class of compounds according to the invention are those of formula (1a) wherein $R_2$ is a hydroxy hydroxymethyl, phenylthio, methylthio or methylsulphinyl, and $R_1$ is pivaloyloxymethyl, 1-pivaloyloxyethyl, acetoxymethyl, 1-acetoxyethyl, 1-methoxy-1-methylethylcarbonyloxymethyl, 1-(1-methoxy-1-methylethylcarbonyloxyethyl), 1-benzoyloxyethyl, 1-isopropoxycarbonyloxyethyl, cyclohexyloxycarbonyloxymethyl, 1-(4-ethylcyclohexyloxycarbonyloxyethyl or more particularly 1-cyclohexyloxycarbonyloxyethyl group. Particularly preferred compounds from within this class are those wherein $R_2$ represents methylthio or methylsulphinyl.

Specific preferred compounds according to the invention include esters of (4S, 8S, 9R, 10S, 12R)-4-methylthio-10-(1-hydroxyethyl)-11-oxo-1-azatricyclo[7.2.0.0.$^{3,8}$.] undec-2-ene-2 carboxylic acid such as the pivaloyloxymethyl, 1-pivaloyloxyethyl, acetoxymethyl, 1-acetoxyethyl, 1-methoxy-1-methylethylcarbonyloxymethyl, 1-(1-methoxy-1-methylethylcarbonyloxyethyl), 1-benzoyloxyethyl,1-isopropoxycarbonyloxyethyl, cyclohexyloxycarbonyloxymethyl, 1-(4-ethylcyclohexyloxycarbonyloxyethyl or more particularly 1-cyclohexyloxycarbonyloxyethyl ester.

Compounds according to the invention when administered orally, exhibit a broad spectrum of antibacterial activity against a wide range of pathogenic microorganisms. Also they have a very high resistance to all β-lactamases. Compounds of the invention are also relatively stable to renal dehydropeptidase.

Compounds of the invention have been found to exhibit useful levels of activity against strains of Staphylococcus aureus, Streptococcus faecalis, Escherichia coli, Pseudomonas aeruginosa, Clostridium perfringens and Bacteriodes fragilis.

The compounds of the invention may therefore be used for treating a variety of diseases caused by pathogenic bacteria in human beings and animals.

Thus, according to another aspect of the present invention, we provide a compound of formula (I) for use in the therapy or prophylaxis of systemic bacterial infections in a human or animal subject.

According to a further aspect of the invention we provide the use of a compound of formula (I) for the manufacture of a therapeutic agent for the treatment of systemic bacterial infections in human beings and animals.

According to a yet further aspect of the invention we provide a method of treatment of the human or non-human animal body to combat bacterial infections which method comprises administering to the body

an effective amount of a compound of formula (I).

It will be appreciated by those skilled in the art that reference herein to treatment extends to prophylaxis as well as the treatment of established infections or symptoms.

It will further be appreciated that the amount of a compound of the invention required for use in treatment will vary with the nature of the condition being treated and the age and the condition of the patient and will be ultimately at the discretion of the attendant physician or veterinarian. In general however doses employed for adult human treatment will typically be in the range of 200-2000mg per day e.g. 1000mg per day.

The desired dose may conveniently be presented in a single dose or as divided doses administered at appropriate intervals, for exmaple as two, three, four or more sub-doses per day.

While it is possible that, for use in therapy, a compound of the invention may be administered as the raw chemical it is preferable to present the active ingredient as a pharmaceutical formulation.

The invention thus further provides a pharmaceutical formulation for oral administration comprising a compound of formula (I) or a pharmaceutically acceptable acid addition salt thereof together with one or more pharmaceutically acceptable carriers therefor and, optionally, other therapeutic and/or prophylactic ingredients. The carrier(s) must be 'acceptable' in the sense of being compatible with the other ingredients of the formulation and not deleterious to the recipient thereof.

The pharmaceutical compositions according to the invention may take the form of, for example tablets or capsules prepared by conventional means with pharmaceutically acceptable excipients such as binding agents (e.g. pregelantinised maize starch, polyvinylpyrrolidone or hydroxypropylmethylcellulose), fillers (e.g. starch, lactose, micro-crystalline cellulose or calcium phosphates), lubricants (e.g. magnesium stearate, hydrogenated vegatable oils, talc, silica, polyethyleneglycols), disintegrants (e.g. potato starch or sodium starch glycloate), or wetting agents (e.g. sodium lauryl sulphate). Flow aids e.g. silicon dioxide may also be used if desired. The tablets may be coated by methods well know in the art.

Liquid preparations for oral administration may take the form of, for example, solutions, syrups or suspensions, or they may be presented as a dry product either for consitution with water or other suitable vehicle before use for administration as a liquid or for direct administration and then washed down with water or other suitable liquid. Such liquid preprations may be prepared by conventional means with pharmaceutically accepable additives such as suspending agents (e.g. sorbitol syrup, methyl cellulose or hydrogenated edible fats and oils such as hydrogenated castor oil), emulsifyng or thickening agents (e.g. lecithin, aluminium stearates or acacia), non-aqueous vehicles (e.g. almond oil, fractionated coconut oil, oily esters or ethyl alcohol), preservatives (e.g. methyl or butyl p-hydroxybenzoates or sorbic acid) and suitable flavouring and sweetening agents.

Compounds of formula (I) may be prepared by esterification of the carboxylic acid (II)

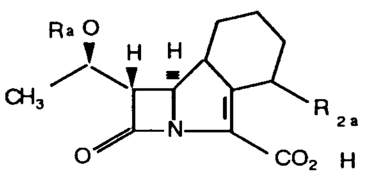

(II)

in which $R_a$ is hydrogen or a hydroxyl protecting group and $R_{2a}$ has the meanings defined for $R_2$ in formula (I) or is a protected derivative thereof; or a reactive derivative thereof and if required or desired subjecting the resulting compound prior to or subsequent to any separation into its sterochemical isomers, to removal of one or more protecting groups. When $R_a$ represents an hydroxyl protecting groups this may be for example a hydrocarbyl silyl group such as trialkylsilyl e.g. trimethylsilyl or t-butyldimethysilyl.

The esterification of a compound of formula (II) may be carried out by reaction with a compound $R_1X$ in which $R_1$ has the meanings defined above in formula (I) and X is a leaving group such a halogen e.g. chlorine, bromine or iodine, or an alkyl or aryl sulphonate such as mesylate or tosylate, in the presence of a base. The reaction is preferably effected in the presence of a solvent, the nature of which is not critical, provided that it has no adverse effect upon the reaction. Suitable solvents include dimethylformamide, dimethylacetamide, or dimethylsulphoxide.

In one embodiment of this process the reaction is conveniently carried out using a salt such as an alkali metal salt e.g. potassium or sodium salt of the carboxylic acid (II) in a polar solvent such as dimethylformamide and optionally in the presence of tetrabutylammonium bromide.

The esterification reaction may be conveniently carried out using a compound of formula (II) in which $R_a$ represents a hydrogen atom. If the esterfication reaction is carried out on a compound of formula (II) in

which $R_a$ represents a hydroxyl protecting group and or $R_{2a}$ is a protected hydroxyl or protected hydroxymethyl group then the hydroxy protecting group may be removed by conventional procedures. For example when $R_a$ and the hydroxy protecting group in $R_{2a}$ is a tert butyldimethylsilyl group this may be removed by treatment with tetrabutylammonium fluoride and acetic acid.

The compounds of formula (II) may be prepared by known methods e.g. as described in EP-A-0416963.

In the formulae (I) and (II) shown above when there is an asymmetric carbon atom and no specific configuration is shown then the formulae include all possible configurations.

Specific stereoisomers of the compounds of formula (I) and as defined in formulae 1a, 1b, 1c and 1d, essentially free of the other stereoisomers may be prepared by using the general processes described above starting with the appropriate stereoisomer of formula (II).

In order that the invention may be more fully understood the following examples are given by way of illustration only.

In the Examples, unless otherwise stated:

Melting points (m.p.) were determined on a Gallenkamp m.p. apparatus and are uncorrected. All temperatures refer to $^0C$.

Infrared spectra were measured in chloroform-d1 solutions on a FT-IR instrument. Proton Magnetic Resonance (1H-NMR) spectra were recorded at 300 MHz as solutions in chloroform-$d_1$. Chemical shifts are reported in ppm downfield ($\delta$) from $Me_4Si$, used as an internal standard.

Example 1

1-(Cyclohexyloxycarbonyloxy)-ethyl(4S,8S,9R,10S,12R)-4-methylthio-10-(1-hydroxyethyl)-11-oxo-1-azatricyclo [7.2.0.$0^{3,8}$]undec-2-ene-2-carboxylate

A solution of potassium (4S,8S,9R,10S,12R)-4-methylthio-10-(1-hydroxyethyl)-11-oxo-1-azatricyclo [7.2.0.03,8]undec-2-ene-2-carboxylate (0.270g) in dry N,N-dimethylformamide (20ml) was added dropwise under nitrogen at -$10^0$, to a stirred solution of cyclohexyl 1-bromoethylcarbonate (0.319g in N,N-dimethylformamide (10ml) and potassium carbonate (0.189g). The resulting mixture was stirred at -$10^0$ for three hours and then concentrated in vacuum. Ethyl acetate (50ml) was added to the solution, which was then washed with water (30ml) and dried over sodium sulfate. The solvent was removed under vacuum and the residue (520mg) was purified by column chromatography on silica gel, using cyclohexane/ ethylacetate 7/3, to obtain after evaporation of the solvent the title compound as a white solid, 0.22g;m.p 45-$65^0$; t.l.c. cyclohexane/ethylacetate 1/1 $R_f$ = 0.64; IR ($CDCl_3$), $V_{max}$($Cm^{-1}$): 1776(c=0), 1765 (c=o), 1626(c=c), 1620(c=c);

$^1$H-NMR(300 MHz, $CDCl_3$): 6.887(q), 4.72(bs), 4.64(m), 4.3-4.18(m), 3.481(m), 3.242(d.d), 2.013(s), 2.005(s), 2.06-1.98(m), 1.98-1.85(m), 1./85-1.7(m), 1.702(d), 1.604(d), 1.586(d), 1.6-1.3(m), 1.314(d), 1.299(d). ppm.

Pharmacy Examples

| Tablets | |
|---|---|
| | mg/tab |
| Compound of Example 1 | 320 |
| Lactose | 150 |
| Ethyl cellulose | 20 |
| Sodium Lauryl sulphate | 7 |
| Magnesium stearate | 3 |
| Tablet core | 500mg |

The active ingredient and the lactose are blended together and then granulated using water as the granulation fluid. The dried granules are blended with ethyl cellulose, sodium lauryl sulphate and magnesium stearate and the tablet core formed using an appropriate punch. The tablet may then be coated using conventional techniques and coatings.

| Granules | |
|---|---|
| | mg/unit dose |
| Compound of Example 1 | 320 |
| Starch | 100 |
| Cellulose | 40 |
| Polymethacrylate | 30 |
| Sodium lauryl sulphate | 7 |
| Magnesium stearate | 3 |
| Flavouring agent | qs |

A solution of the active ingredient in ethanol is sprayed into a suitable fluid bed granulator charged with the major excipients. The granules so formed are dried and screened. If desired the granules may then be coated with a suitable enteric coating and dried. The dried granules are then blended with the remaining excipients including any flavouring agent and coated, for example with an enteric coating. The granules thus obtained may be filled into capsules or the like for a single dose presentation or filled into bottles for subsequent preparation of a multi dose oral liquid presentation.

The antibacterial activity of the compounds of the invention may be determined by oral administration to mice in a conventional protection test.

The compounds of the invention are essentially non-toxic at therapeutically useful dose levels.

## Claims

**1.** Compounds of formula (I)

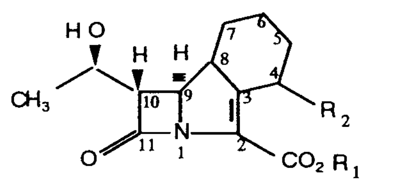

(I)

in which $R_1$ represents the group

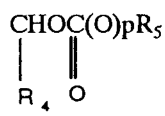

wherein $R_4$ represents a hydrogen atom or a $C_{1-4}$alkyl group; p is zero or one, and $R_5$ represents a group selected from $C_{1-6}$alkyl, $C_{5-8}$cycloalkyl optionally substituted by a $C_{1-3}$alkyl group, phenyl, or $C_{1-4}$alkyl substituted by a $C_{1-3}$alkoxy group.

$R_2$ represents a $C_{1-3}$ alkyl, hydroxy, or hydroxymethyl group or $R_2$ represents the group $XR_3$ wherein X is oxygen and $R_3$ represents a $C_{3-7}$ cycloalkyl or phenyl group or X is the group S(O)n wherein n is zero or the integer 1 or 2 and $R_3$ is $C_{1-5}$alkyl, $C_{3-7}$cycloalkyl or phenyl.

**2.** Compounds as claimed in Claim 1 have the configuration

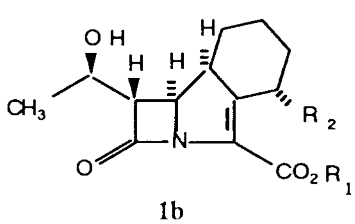

1b

where $R_1$ and $R_2$ are as defined in Claim 1.

**3.** Compounds as claimed in Claim 1 or Claim 2 wherein $R_2$ represents a hydroxy, hydroxymethyl, phenylthio, or $C_{1-5}$alkylthio or $C_{1-5}$alkylsulphinyl group.

**4.** Compounds as claimed in any of Claims 1 to 3 wherein $R_2$ is a methylthio or methylsulphinyl group.

**5.** Compounds as claimed in any of Claims 1 to 4 wherein $R_4$ is a hydrogen atom or a methyl group.

**6.** Compounds as claimed in ahy of Claims 1 to 5 wherein $R_5$ represents a methyl, ethyl, isopropyl, t-butyl, -1-methoxy-1-methyl, ethyl, phenyl, cyclohexyl or 4-ethylcyclohexyl group.

**7.** Compounds as claimed in any of Claims 1 to 6 wherein $R_2$ is hydroxy, hydroxymethyl, phenylthio, methylthio or methylsulphinyl, and $R_1$ is pivaloyloxymethyl, 1-pivaloyloxyethyl, acetoxymethyl, 1-acetoxyethyl, 1-methoxy-1-methylethylcarbonyloxymethyl, 1-(1-methoxy-1-methylethylcarbonyloxyethyl), 1-benzoyloxyethyl, 1-isopropoxycarbonyloxyethyl, cyclohexyloxycarbonyloxymethyl, 1-(4-ethylcyclohexyloxycarbonyloxyethyl or 1-cyclohexyloxycarbonyloxyethyl.

**8.** Compounds as claimed in any of Claims 1 to 7 where $R_2$ is methylthio or methylsulphinyl.

**9.** The pivaloyloxymethyl, 1-pivaloyloxyethyl, acetoxymethyl, 1-acetoxyethyl, 1-methoxy-1-methylethylcarbonyloxymethyl, 1-(1-methoxy-1-methylethylcarbonyloxyethyl), 1-benzoyloxyethyl, 1-isopropoxycarbonyloxyethyl, cyclohexyloxycarbonyloxymethyl, 1-(4-ethylcyclohexyloxycarbonyloxyethyl or 1-cyclohexyloxycarbonyloxyethyl esters of (4S, 8S, 9R, 10S, 12R)-4-methylthio-10-(1-hydroxyethyl)-11-oxo-1-azatricyclo[7.2.0.0.$^{3}$.$^{8}$.] undec-2-ene-2 carboxylic acid.

**10.** 1-Cyclohexyloxycarbonyloxethyl (4S,8S,9R,10S,12R) -4-methylthio-10-(1-hydroxyethyl)-11-oxo-1-azatricyclo[7.2.0.0.$^{3.8}$]undec-2-ene-2-carboxylate.

**11.** Compounds as claimed in any of Claims 1 to 10 for use in therapy or prophylaxis of systemic bacterial infections in a human or animal subject.

**12.** The use of a compound as claimed in any of Claims 1 to 10 in the manufacture of a therapetuc agent for the treatment or prophylaxis of systemic bacterial infections in a human or animal body.

**13.** Pharmaceutical compositions comprising a compound as claimed in any of Claims 1 to 10 in admixture with one or more physiologically acceptable carriers or excipients.

**14.** A method of treatment of a human or non-human body to combat bacterial infections comprising administration to said body of an effective amount of a compound as claimed in any of Claims 1 to 10.

**15.** A process for the preparation of compounds of general formula (I) as defined in Claim 1 which comprises either (a) reacting a compound of general formula (II).

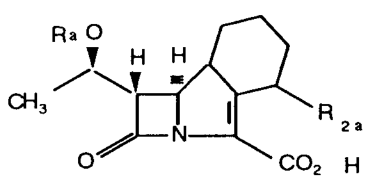

(II)

(where $R_a$ represents a hydrogen atom or a hydroxyl protecting group and $R_{2a}$ has the meanings defined for $R_2$ in Claim 1 or is a protected derivative thereof), or a salt or reactive derivative thereof with an esterifying agent serving to introduce a group $R_1$ as defined in Claim 1, and thereafter and if necessary or desired reacting the product to replace a hydroxyl protecting group $R_a$ by hydrogen and or converting the group $R_{2a}$ into a group $R_2$ as defined in Claim 1.

European Patent Office

# PARTIAL EUROPEAN SEARCH REPORT

which under Rule 45 of the European Patent Convention shall be considered, for the purposes of subsequent proceedings, as the European search report

Application Number

EP 92 10 3594

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | US-A-4 374 848 (B.G. CHRISTENSEN)<br>* Whole document *<br>--- | 1,11-14 | C 07 D 477/00<br>A 61 K 31/40 |
| P,A | EP-A-0 422 596 (TAKEDA CHEMICAL INDUSTRIES, LTD)<br>* Examples 10,11,29,32 and claims *<br>--- | 1,11-14 | |
| D,P A | EP-A-0 416 953 (GLAXO S.P.A.)<br>* Claims *<br>----- | 1,11-14 | |

TECHNICAL FIELDS SEARCHED (Int. Cl.5)

C 07 D
A 61 K

## INCOMPLETE SEARCH

The Search Division considers that the present European patent application does not comply with the provisions of the European Patent Convention to such an extent that it is not possible to carry out a meaningful search into the state of the art on the basis of some of the claims

Claims searched completely :

Claims searched incompletely :

Claims not searched :

Reason for the limitation of the search:

Remark: Although claim 14 is directed to a method of treatment of (diagnostic method practised on) the human/animal body (Article 52(4) EPC) the search has been carried out and based on the alleged effects of the compound/composition

| Place of search | Date of completion of the search | Examiner |
|---|---|---|
| THE HAGUE | 18-05-1992 | CHOULY J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document